# EUROPEAN PATENT APPLICATION

(11) **EP 4 353 168 A1**
(43) Date of publication of application: **17.04.2024**
(21) Application number: 22201068.8
(22) Date of filing: 12.10.2022
(51) Int. Cl.: A61B 17/80, A61F 2/28, A61F 2/30, A61F 2/44, B33Y 80/00

(54) **3D PRINTED SEMI-FINISHED PART FOR MEDICAL IMPLANT MANUFACTURING AND MEDICAL IMPLANT THEREOF**

(71) Applicant: Kumovis GmbH, 81369 München (DE)
(72) Inventor: BRUYAS, Arnaud, 81549 München (DE); KRIEGER, Yannick, 81541 München (DE); LEONHARDT, Stefan, 81677 München (DE); PAMMER, Sebastian, 81677 München (DE)
(74) Representative: DTS Patent- und Rechtsanwälte Schnekenbühl und Partner mbB

(57) **Abstract**

The present invention relates to a 3D-printed semi-finished part (100) for medical implant manufacturing, the semi-finished part (100) including:
at least one functional feature (10) provided at a predefined location of the semi-finished part (100).

## Description

The present invention belongs to the field of 3D-printed implants for medical applications.

Medical implants obtained through 3D printing processing are known in the art and have gained increasing importance in the last decades.

For example, WO2021094624A1 discloses an orthopedic implant obtained through a 3D printing process, said orthopedic implant comprising at least one first portion and at least one second portion, the first portion forming a support structure and providing stiffness and mechanical strength to the implant, and the second portion being at least partially made of a biodegradable material to allow improved ingrowth.

A 3D printing device and method for manufacturing a medical implant is known, e.g., from DE102020126764A1.

In particular, fused filament fabrication (FFF) has proven advantageous in manufacturing of medical implants, as it allows to improve osseointegrative effect and primary stability of the implant.

3D-printed medical implants can be manufactured by using semi-finished parts.

In particular, said semi-finished parts are extruded pieces (e.g., made of a high-performance polymer) that are used to define the outline of the implant.

For instance, semi-finished parts may be in the form of a cube, parallelepiped or cylinder. Alternatively, more complex configurations are also possible.

Several medical implants, even with slight variations (e.g., in shape or the like), can be post-processed starting from a single semi-finished part.

3D-printed medical implants quality (e.g., distortion, tolerances, strength, toughness etc.) and specific properties (e.g., microstructure or surface properties) is increasingly in the focus of many scientific investigations.

In particular, there is the need for an improved medical implant having enhanced properties (e.g., improved osseointegration, improved osseo conductivity, improved mechanical properties or the like).

It is an object of the present invention to provide a solution allowing to obtain a 3D-printed medical implant having enhanced properties, e.g. in terms of improved osseointegration, improved osseo conductivity, improved bone replacement and/or augmentation mechanical properties or the like.

This object is achieved by the provision of a 3D-printed semi-finished part according to claim 1.

According to the invention, a 3D-printed semi-finished part for medical implant manufacturing includes:
at least one functional feature provided at a predefined location of the semi-finished part.

The present invention provides a 3D-printed semi-finished part for medical implant manufacturing.

The semi-finished part includes at least one functional feature provided at a predefined location of the semi-finished part.

In particular, the location of the at least one functional feature is defined based on the desired properties of the medical implant to be manufactured.

A plurality of medical implants, even with slight variations (e.g., in shape or the like), can be post-processed from the 3D-printed semi-finished part.

Further, the 3D-printed semi-finished part can be easily and reliably clamped to a computer numerical control (CNC) machine for implementing a 3D printing process for manufacturing the medical instrument.

The medical implant can be manufactured by implementing conventional 3D printing processes that are known in the art.

The invention is based on the basic idea that, by the provision of at least one functional feature at a predefined location of a 3D-printed semi-finished part, it is possible to obtain an improved 3D-printed medical implant having enhanced properties (e.g., improved osseointegration, improved osseo conductivity, improved mechanical properties or the like) that cannot be achieved by implementing conventional 3D printing processes alone.

Advantageously, the at least one functional feature may include a porous structure.

By providing at least one porous structure at a predefined location of the semi-finished part, a plurality of pores can be easily formed on one or more desired locations of the medical implant with high precision.

Advantageously, the at least one functional feature can be made of a material that is different than the material of the semi-finished part.

This allows to provide enhanced strength to selected parts of the semi-finished part and/or shape its cosmetic appearance.

In particular, the semi-finished part can be manufactured through fused filament fabrication (FFF).

This allows to obtain a semi-finished part having improved quality.

Also, the present invention provides a 3D-printed medical implant obtained by using a 3D-printed semi-finished part as defined above.

Advantageously, the medical implant includes at least one porous structure.

The porous structure may be configured and adapted to improve osseointegration of the medical implant after implantation at a target site within the body of a patient.

For this purpose, pores of the at least one porous structure may have a pore size between 0,05 mm and 1,0 mm.

The pores may have a circular shape.

Alternatively, the pores may have a rectangular shape.

Alternatively, the pores may have a gyroid shape.

Alternatively, the pores may have a diamond shape.

Alternatively, the pores may have a Schwarz triangle shape.

The pores size and shape are selected based on the desired properties of the medical implant to be manufactured.

The pores of the at least one porous structure may be interconnected.

This allows to further improve osseointegration of the medical implant after implantation at the target site.

Also, the at least one porous structure may be configured and adapted to influence and/or improve mechanical properties of the medical implant.

For this purpose, the at least one porous structure may include at least one portion acting as a spring.

This allows to obtain a medical implant having compliant mechanics, e.g. allowing micro movements to a certain extent.

Also, the at least one porous structure may include at least one portion having non-linear mechanical properties.

This also allows to obtain a medical implant having compliant mechanics.

Also, the at least one porous structure may include at least one portion having elastic properties.

This allows for elastic deformation of the implant after implantation, which enables a certain degree of freedom of movement for the patient.

The at least one porous structure can be provided on one or more surfaces of the implant.

This is particularly advantageous in case the at least one porous structure is provided to improve osseointegration.

Advantageously, the 3D-printed medical implant may include a plurality of different materials.

Accordingly, it is possible to better tune the properties of the medical implant based on its medical application.

In particular, the medical implant may include at least one high-performance polymer.

In particular, the at least one high-performance polymer may be selected from:
Polyether ether ketone (PEEK);
Polyetherketoneketone (PEKK);
Polyphenylsulfone (PPSU);
Polyaryletherketone (PAEK);
Polyetherketone (PEK);
Polyamide-imide (PAI), or
Polyethylenimine (PEI);

Also, the medical implant may include at least one high-performance polymer comprising one or more ceramic fillers.

For instance, biphasic calcium phosphate (BCP) or bioactive glasses can be used as the ceramic filler.

By the provision of one or more ceramic fillers, it is possible to improve osseo conductivity and osseointegration.

Also, the medical implant may include at least one high-performance polymer comprising one or more fiber reinforcement materials.

By the provision of one or more fiber reinforcement materials at predefined locations of the medical implant, it is possible to better tune the mechanical properties of the medical implant according to its medical application.

Also, the medical implant may include at least one high-performance polymer filled with a radio opaque filler.

By the provision of a radio opaque filler, predefined parts of the medical implant can be made visible in X-Ray/CT.

Also, the medical implant may include at least one drug-loaded material.

By the provision of a drug-loaded material, it is possible to provide area-specific drug delivery to a predefined target area.

Advantageously, the drug may include an anti-inflammatory agent.

Also, the drug may include an antibiotic agent.

The 3D-printed medical implant can be a spinal fusion device, e.g. a spinal cage.

Also, the 3D-printed medical implant can be an osteotomy wedge.

Also, the 3D-printed medical implant can be a total endoprosthesis, e.g. for a knee, hip, shoulder or the like.

Also, the 3D-printed medical implant can be a bone fixation plate.

Also, the 3D-printed medical implant can be a Cranio Maxillo Facial (CMF) implant, e.g. a cranial implant or onlay.

Further details and advantages of the present invention shall now be disclosed in connection with the drawings, where:

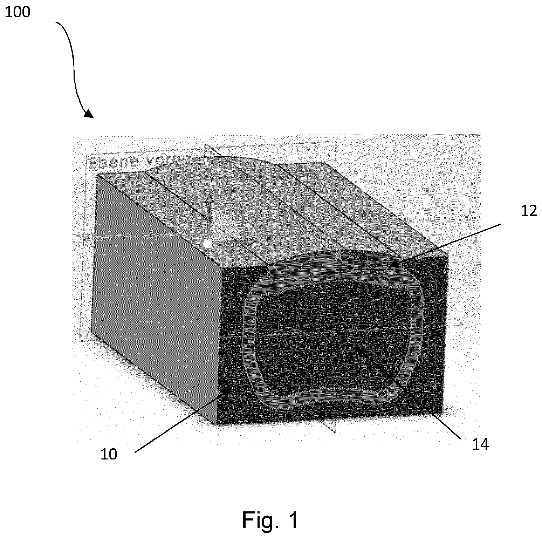

**Fig. 1** is a perspective view of a 3D-printed semi-finished part according to an embodiment of the invention, in particular for manufacturing a spinal cage.

**Fig. 1** shows a 3D-printed semi-finished part 100 for medical implant manufacturing according to an embodiment of the invention.

In the present embodiment, the semi-finished part 100 is configured and adapted for manufacturing a spinal cage (not shown).

The semi-finished part 100 includes a functional feature 10.

Not shown is that the semi-finished part 100 may also include additional functional features, provided at predefined locations of the semi-finished part 100.

In the shown embodiment, the functional feature 10 includes a porous structure 10.

The porous structure 10 is made of a porous material, different than the material of the semi-finished part 100.

In the shown embodiment, the semi-finished part 100 is made of a non-porous material 12.

The porous structure 10 is arranged to surround the non-porous material 12 of the semi-finished part 100.

Further, the porous structure 10 is also arranged to fill a cavity 14 defined by the non-porous material 12 of the semi-finished part 100.

Advantageously, the semi-finished part 100 is manufactured through fused filament fabrication (FFF).

FFF is a 3D printing process which is well-known in the art and which is therefore not further described for the sake of brevity.

The present invention also provides a 3D-printed medical implant (now shown).

The 3D-printed medical implant can be a spinal fusion device such as a spinal cage or the like.

Alternatively, the 3D-printed medical implant can be an osteotomy wedge.

As a further alternative, the 3D-printed medical implant can be a bone fixation plate.

Also, the 3D-printed medical implant can be a total endoprosthesis, e.g. for a knee, hip or shoulder.

According to yet another alternative, the 3D-printed medical implant can be a Cranio Maxillo Facial (CMF) implant, e.g. a cranial implant or onlay.

The medical implant is obtained by using a 3D-printed semi-finished part, such as the semi-finished part 100 describe above.

The semi-finished part includes at least one functional feature provided at a predefined location of the semi-finished part.

In one embodiment, the implant includes at least one porous structure.

The at least one porous structure may be adapted for improving osseointegration of the medical implant after implantation.

In such a case, it is preferable that the at least one porous structure is provided on one or more surfaces of the implant.

The pores of the at least one porous structure have a pore size between 0,05 mm and 1,0 mm.

The pores of the at least one porous structure have a pore shape selected among circular, rectangular, gyroid, diamond or Schwarz triangle.

In the present embodiment, pores of the at least one porous structure are interconnected.

In an alternative configuration, pores of the at least one porous structure may be non-interconnected.

The porous structure may also be configured and adapted to influence and improve mechanical properties of the medical implant.

For this purpose, the at least one porous structure may include at least one portion acting as a spring, thereby allowing to obtain a medical implant with compliant mechanics.

Additionally or alternatively, the at least one porous structure may include at least one portion having non-linear mechanical properties, also allowing to obtain a medical implant with compliant mechanics.

Additionally or alternatively, the at least one porous structure may include at least one portion having elastic properties that allow for elastic deformation of the implant after implantation, thereby enabling a certain degree of freedom of movement for the patient.

In a further embodiment, optionally in combination with one or more features of the embodiment described above, the medical implant includes a plurality of different materials. In particular, the medical implant may include at least one high-performance polymer. Preferably, said high-performance polymer is selected from:
Polyether ether ketone (PEEK);
Polyetherketoneketone (PEKK);
Polyphenylsulfone (PPSU);
Polyaryletherketone (PAEK);
Polyetherketone (PEK);
Polyamide-imide (PAI), or
Polyethylenimine (PEI);

Also, the medical implant may include at least one high-performance polymer comprising one or more ceramic fillers.

For instance, biphasic calcium phosphate (BCP) or bioactive glasses can be used as a ceramic filler.

By the addition of one or more ceramic fillers, it is possible to improve osseo conductivity and osseointegration.

Also, the medical implant may include at least one high-performance polymer comprising one or more fiber reinforcement materials.

By the provision of one or more fiber reinforcement materials at predefined locations of the medical implant, it is possible to better tune the mechanical properties of the medical implant according to its medical application.

Also, the medical implant may include at least one high-performance polymer filled with a radio opaque filler.

By the provision of a radio opaque filler, predefined parts of the medical implant can be made visible in X-Ray/CT.

Also, the medical implant may include at least one drug-loaded material.

By the provision of a drug-loaded material, it is possible to provide area-specific drug delivery to a predefined target area.

Advantageously, the drug may include an anti-inflammatory agent.

Also, the drug may include an antibiotic agent.

### References

- 100: 3D-printed semi-finished part
- 10: Functional feature (porous structure)
- 12: Non-porous material
- 14: Cavity

## Claims

1. A 3D-printed semi-finished part (100) for medical implant manufacturing, the semi-finished part (100) including:
at least one functional feature (10) provided at a predefined location of the semi-finished part (100).

2. The 3D-printed semi-finished part (100) according to claim 1,
**characterized in that** the at least one functional feature (10) includes a porous structure.

3. The 3D-printed semi-finished part (100) according to claim 1 or 2,
**characterized in that** the at least one functional feature (10) is made of a material that is different than the material of the semi-finished part (100).

4. The 3D-printed semi-finished part (100) according to any one of the preceding claims,
**characterized in that** the semi-finished part (100) is manufactured through fused filament fabrication (FFF).

5. A 3D-printed medical implant obtained by using a 3D-printed semi-finished part (100) according to any one of claims 1 to 4.

6. The 3D-printed medical implant according to claim 5,
**characterized in that** the medical implant includes at least one porous structure.

7. The 3D-printed medical implant according to claim 6,
**characterized in that** pores of the at least one porous structure have a pore size between 0,05 mm and 1,0 mm.

8. The 3D-printed medical implant according to claim 6 or 7,
**characterized in that** pores of the at least one porous structure have a pore shape selected among:
circular;
rectangular;
gyroid;
diamond, or
Schwarz triangle.

9. The 3D-printed medical implant according to any one of claims 5 to 8,
**characterized in that** pores of the at least one porous structure are interconnected.

10. The 3D-printed medical implant according to any one of claims 5 to 9,
**characterized in that** the at least one porous structure includes one or more among:
at least one portion acting as a spring;
at least one portion having non-linear mechanical properties, and/or
at least one portion having elastic properties that allow for elastic deformation of the implant after implantation.

11. The 3D-printed medical implant according to any one of claims 5 to 10,
**characterized in that** the at least one porous structure is provided on one or more surfaces of the implant.

12. The 3D-printed medical implant according to any one of claims 5 to 11,
**characterized in that** the medical implant includes a plurality of different materials.

13. The 3D-printed medical implant according to claim 12,
**characterized in that** said plurality of different materials is selected from:
at least one high-performance polymer, preferably wherein said high-performance polymer is selected from:
Polyether ether ketone (PEEK);
Polyetherketoneketone (PEKK);
Polyphenylsulfone (PPSU);
Polyaryletherketone (PAEK);
Polyetherketone (PEK);
Polyamide-imide (PAI), or
Polyethylenimine (PEI);
at least one high-performance polymer comprising one or more ceramic fillers, preferably wherein the one or more ceramic fillers include biphasic calcium phosphate (BCP) or bioactive glasses;
at least one high-performance polymer comprising one or more fiber reinforcement materials;
at least one high-performance polymer filled with a radio opaque filler, and/or
at least one drug-loaded material, preferably wherein said drug includes an anti-inflammatory agent and/or an antibiotic agent.

14. The 3D-printed medical implant according to any one of claims 5 to 13,
**characterized in that** the medical implant is one among:
a spinal fusion device,
an osteotomy wedge;
a total endoprosthesis;
a bone fixation plate, or
a Cranio Maxillo Facial (CMF) implant.
